# EUROPEAN PATENT SPECIFICATION

(11) **EP 0 708 336 B1**
(45) Date of publication and mention of the grant of the patent: **06.08.2003**
(21) Application number: 95306389.8
(22) Date of filing: 12.09.1995
(51) Int. Cl.: G01N 33/566, G01N 15/14, G01N 33/577, G01N 33/58

(54) **Method to distinguish granulomonocytic progenitors in a population of CD34+ cells**
Verfahren zur Unterscheidung von granulomoncytischen Progenitoren in einer Population von CD34+ Zellen
Méthode de distinguer les progéniteurs granulomonocytiques dans une population de cellules CD34+

(30) Priority: 23.09.1994 US 311690
(43) Date of publication of application: 24.04.1996
(73) Proprietor: Becton Dickinson and Company, Franklin Lakes, New Jersey 07417-1880 (US)
(72) Inventor: Olweus, Johanna, Fremont, California (US); Lund-Johansen, Fridtjof, PaloAlto, California 94306 (US); Terstappen, Leon, Huntingdon Valley, Philadelphia (US)
(74) Representative: Ruffles, Graham Keith

(56) References cited:
- EP-A- 0 455 482
- EP-A- 0 610 774
- CHEMICAL ABSTRACTS, vol. 118, no. 21, 24 May 1993 Columbus, Ohio, US; abstract no. 211105, J. M. KERST ET AL.: "Granulocyte colony-stimulatig factor induces hFc-gamma-RI (CD64 antigen)- positive neutrophils via an effect on myeloid precursor cells. " page 725; column 2; XP002029597 & BLOOD, vol. 81, no. 6, 1993, pages 1457-1464,
- CHEMICAL ABSTRACTS, vol. 119, no. 21, 22 November 1993 Columbus, Ohio, US; abstract no. 223637, S. K. SALGAR ET AL.: "Enzyme-linked immunosorbent assay and flow cytometric methods to screen hybridoma culture supernatants for antibodies to bovine neutrophil surface antigens, and monoclonal antibody production and characterization. " page 778; column 2; XP002029598 & AM. J. VET. RES., vol. 54, no. 9, 1993, pages 1415-1425,
- CHEMICAL ABSTRACTS, vol. 115, no. 7, 19 August 1991 Columbus, Ohio, US; abstract no. 69689, H. B. FLEIT ET AL.: "The human monocyte-like cell line THP-1 expresses Fc-gamma-RI and Fc-gamma-RII" page 617; column 1; XP002029599 & J. LEUKOCYTE BIOL., vol. 49, no. 6, 1991, pages 556-565,
- JOURNAL OF IMMUNOLOGICAL METHODS, vol. 162, no. 1, 4 June 1993, NEW YORK US, pages 17-21, XP002029596 K. TAKAHASHI ET AL.: "New flow cytometric method for surface phenotyping basophils from peripheral blood."
- CHEMICAL ABSTRACTS, vol. 117, no. 9, 31 August 1992 Columbus, Ohio, US; abstract no. 88227, P. RUIZ ET AL.: "Granulocyte Fc-gamma receptor recognition of cell bound and aggregated IgG: effect of gamma-interferon." page 580; column 2; XP002029600 & AM. J. HEMATOL., vol. 39, no. 4, 1992, pages 257-263,
- CHEMICAL ABSTRACTS, vol. 120, no. 3, 17 January 1994 Columbus, Ohio, US; abstract no. 29315, M. NOTTER ET AL.: "Selective targeting of human lymphokine-activated killer cells by CD3 monoclonal antibody against the interferon-inducible high-affinity Ec-gamma-RI receptor (CD64) on autologous acute myeloid leukemic blast cells." page 715; column 1; XP002029601 & BLOOD, vol. 82, no. 10, 1993, pages 3113-3124,
- CHEMICAL ABSTRACTS, vol. 115, no. 11, 16 September 1991 Columbus, Ohio, US; abstract no. 112365, G. C. KINDT ET AL.: "Identification and structural characterization of Fc-gamma-receptors on pulmonary alveolar macrophages." page 704; column 1; XP002029602 & AM. J. PHYSIOL. , vol. 260, no. 6, pt. 1, 1991, pages L403-L411,
- KERST JM ET AL: "", BLOOD, , 15. March 1993, vol. 81, no. 6, pages 1457 to 1464

## Description

### Background of Invention

Pluripotent hematopoietic stem cells have the ability to proliferate and differentiate into various lineage-committed progenitor cells. These cells further proliferate and mature before entering the blood stream. A variety of factors have been identified that alter the proliferative acivity and differentiation of lineage-committed progentiors and pluripotent progenitors. Knowledge about changes in the antigenic profile associated with lineage-commitment may facilitate studies of these processes.

Hematopoietic progenitor cells are contained within several different organs early in fetal life, whereas they reside mainly in the bone marrow after 3 months of gestation. These cells can be identified by the expression of CD34 and constitute 1-5% of adult bone marrow and 5-12 % of fetal bone marrow cells. Recently, there has been a considerable progress in the characterization of CD34+ cell subsets with different functional capacity and immunophenotype. The majority of pluripotent hematopoietic cells have been shown to be CD34⁺⁺, CD38^{-/lo}, Thy-1⁺ (CDw9O), CD45RA^{lo} and CD71^{lo}. CD34+,HLADR^{-/lo} cells in adult bone marrow have also been reported as enriched for non-committed progenitors. In fetal bone marrow, fetal liver and cord blood, however, these cells express HLADR.

Lineage-commitment is associated with increased levels of the CD38 antigen and lower levels of CD34. The lineage committed cells stain heterogeneously with a number of antibodies. For example, CD 19 is widely accepted as an early B-lymphoid-specific marker. Antigens that have been reported to indicate early myeloid differentiation include CD45RA, CD71, CD13 and CD33. Even though these myeloid markers are useful for separation of certain types of progenitor-subsets, none of them are lineage-restricted. CD45RA is present on both lymphoid and granulo-monocytic progenitors and CD71 is expressed on a large number of non-erythroid bone marrow cells. Two recent reports showing expression of CD13 and CD33 on primitive progenitor cells question the specificity of these markers for myeloid committed cells. It therefore seems necessary to identify additional and more specific myeloid lineage markers for improved purification and studies of hematopoietic progenitor-subsets.

Chem Abs 118: 211105f relates to the expression pattern of CD64 during myelopoiesis.

### Summary of Invention

The instant invention presents an immunophenotypic marker that discriminates myeloid (defined as granulocytic and monocytic) progenitor cells from pluripotent hematopoietic progenitors and cells committed to other lineages. The marker, CD64, is specific for granulomonocytic cells, including their progenitors, and can be used to identify and/or isolate these cells among subsets of lineage-committed progenitors (e.g. CD34⁺,CD38⁺ cells). The CD64 marker is, therefore, more useful for identification and purification of progenitor substrates than CD13 and CD33, which are also present on pluripotent progenitor cells.

In particular, the present invention provides the following methods:

A method of distinguishing granulomonocytic progenitor cells in a population contining CD34+ cells, comprising:
Contacting said cells with antibodies specific for CD34 and CD64; and then flow cytometrically detecting cells that are CD34+CD64.

A method of isolating granulomonocytic progenitor cells from a population containing CD34+ cells, comprising:
Contacting said cells with antibodies specific for CD34 and CD64; and then flow cytometrically isolating cells that are CD34+CD64+.

### Brief Description of the Figures

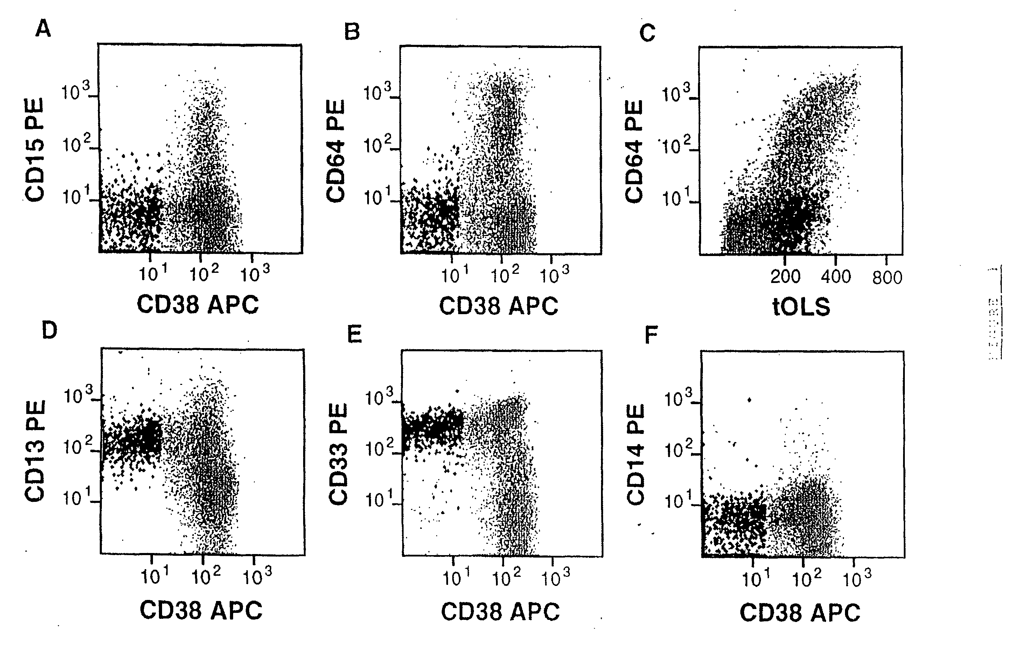

Figure 1 illustrates the staining of CD34+ fetal bone marrow cells with CD15, CD64, CD13, CD33 and CD14. Non-comment and lineage-committed cells were defined and depicted as described in the legend to Figure 1. Labelling with anit-CD14 mAb (panel F) was identical to staining with isotype control (not shown). Note the higher orthogonal light scatter (tOLS) of the CD64+ cells compared to the remaining cells in panel C. The data are taken from one experiment representative of four performed.

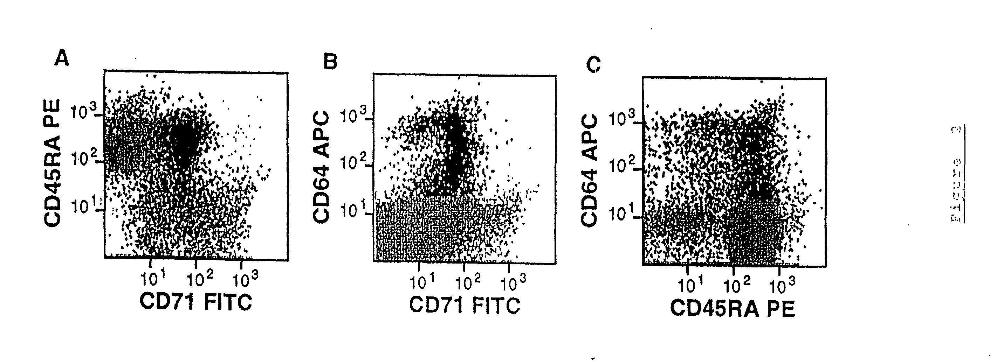

Figure 2 illustrates the phenotypic characterization of CD34+, CD64+ progenitors by costaining with CD45RA and CD71. Panel A, B and C show data for CD34+ FBM cells from a single sample stained with CD34 PerCP, CD64 APC, CD45RA PE and CD71 FITC. The CD64+ cells are depicted black. The majority of these cells are CD45RA+,CD71lo, suggesting that they belong to the granulomonocytic lineage. The remaining CD34+,CD64+ cells are CD45RAlo,CD71lo (panel A and C). The CD64-cells are depicted grey, where the majority represents lymphoid cells (CD45RA+,CD71lo). The remaining grey dots represent erythroid cells (CD45RAlo,CD71++) and non-committed progenitor cells (CD45RAlo,CD71lo). The data are taken from one experiment representative of three performed.

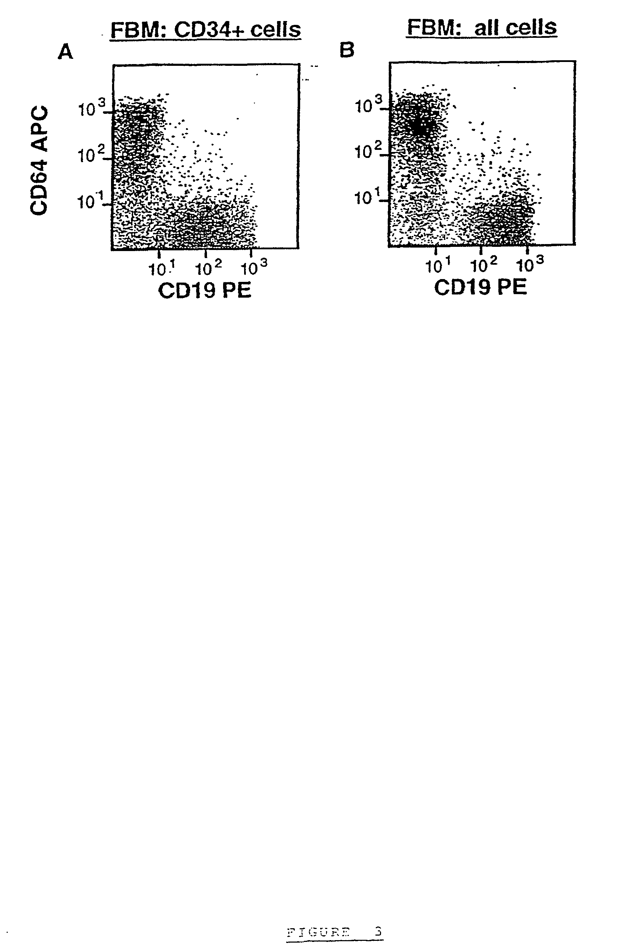

Figure 3 illustrates fetal bone marrow cells stained with CD34 PerCP, CD38 APC, CD19 PE and CD64 APC. Panel C shows CD34+ cells, and panel D represents all bone marrow cells. The CD64+ cells are depicted black. The data are taken from one experiment representative of three performed.

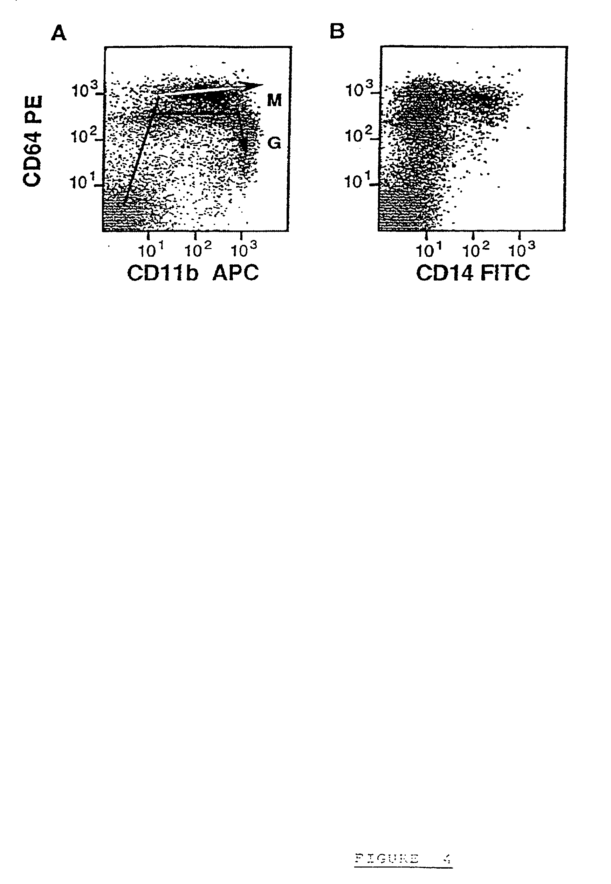

Figure 4 staining of CD64 on differentiating and mature granulo-monocytic cells from fetal bone marrow (FBM). Panel A shows data from a sample of FBM cells stained with CD11b APC, CD64 PE, CD14 FITC and CD34 PerCP. The grey-colored, CD64, CD11b- population contains CD34+ cells as well as cells with light scatter properties of lymphocytes (not shown). The monocytic CD64++,CD14+ cells (M) were painted black in panel B, and their maturation pathway is outlined by the white arrow in panel A. The remaining CD64+ cells are depicted grey. The maturation pathway of the granulocytic CD64+,CD14- cells (G), is outlined by the black arrow in panel B. The data are taken from one experiment representative of four performed

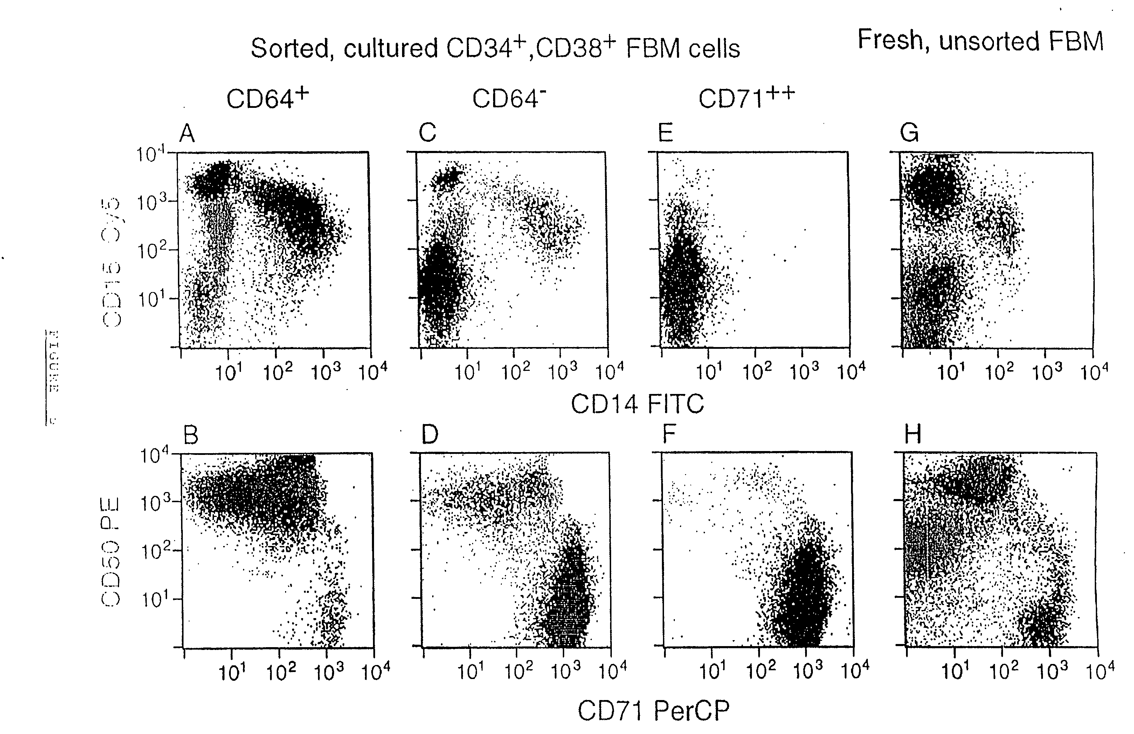

Figure 5 presents flow cytometric analyses of progeny of sorted subsets of CD34+,CD38+ fetal bone marrow (FBM) cells cultured for 6 days (A-F) compared with the analysis of fresh, unsorted FBM (G,H). The sorted subpopulations were CD34+,CD38+,CD64+ (A,B), CD34+,CD38+,CD64- (C,D) and CD34+,CD38+CD71++. The cells from liquid cultures and the fresh FBM cells were stained with CD14 FITC, CD50 PE, CD15 and CD71 biotin; the latter two labeled with anti-mouse 1gM-Cy5 and streptavidin-PerCP, respectively. In the progeny from all the cultured subsets, five subpopulations were distinguished phenotypically and indicated by different colors. Blue: A CD15++,CD50+,CD14-,CD71 dim subset. Yellow: Cells with a similar phenotype, but with lower levels of CD15. Green: CD14+,CD50+,CD71-->+ cells. Light blue: A small population that is CD50+, but negative for the other three markers. Red: CD71++, CD50lo, CD14-, CD15 lo cells. The red, blue and green populations were identified also in freshly isolated FBM. In addition, a population corresponding to Lymphoid cells, with low forward and orthogonal light scatter (CD50+,CD15-,CD14-,CD71-), was identified and depicted grey. The dot plots are taken from a representative experiment (n=6 for A-F, n=3 for G,H). See Figure 5 for corresponding morphology.

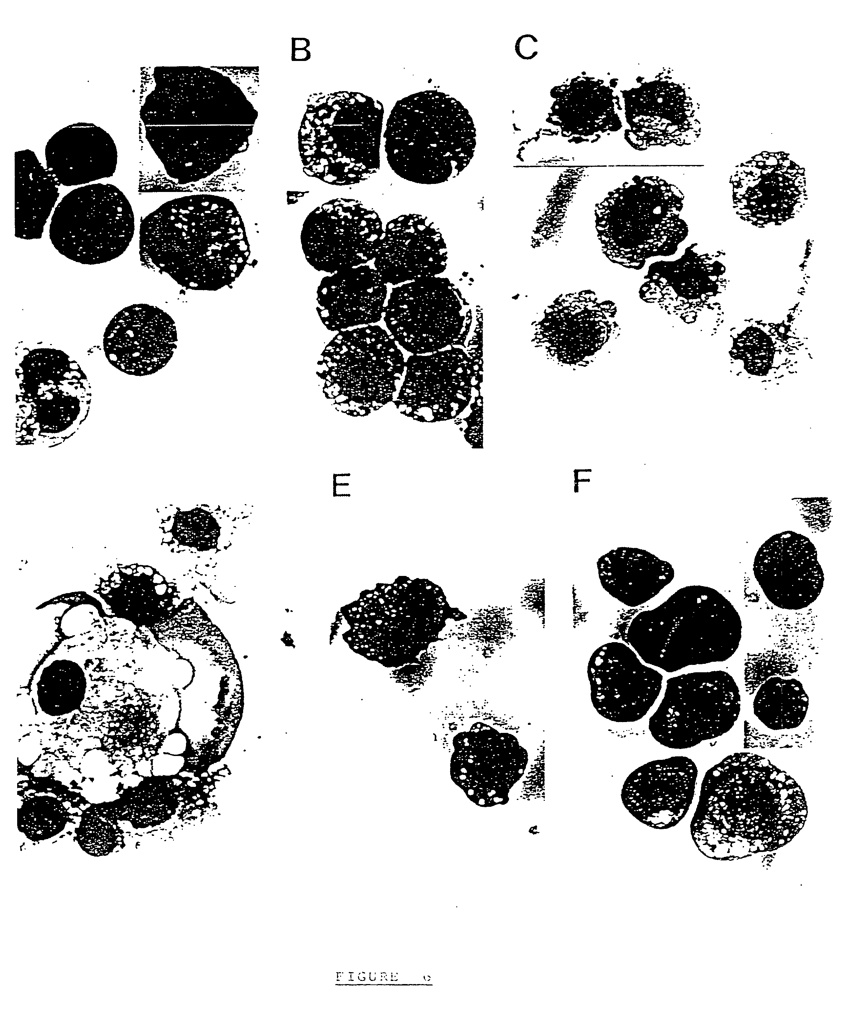

Figure 6 presents photomicrographs of Wright-Giemsa-stained cells obtained from progeny of sorted CD34+,CD38+,CD64+ FBM cells cultured for one week. The five different subpopulations phenotypically defined in Figure 4 were sorted and evaluated morphologically. Panel A shows cells with morphological characteristics of promyelocytes and later stages of neutrophilic granulocytes (blue dots in Figure 4). Panel B displays cells with the morphological appearance of clustered basophilic granulocytes (yellow dots in Figure 4). The cells in Panel C have characteristics of small monocytes/macrophages with fine granules in the cytoplasm (green dots that are CD15++ Figure 5). The macrophage-like cells in Panel D display an abundance of cytoplasmic vacuoles, and one of the large cells frequently found in this population is shown (green dots that are CD15+ in Figure 4). Panel E is taken from the small subset of cells (light blue dots in Figure 4) with coarse, cytoplasmic granules and an irregularly shaped nucleus, indicating that they belong to the granulocytic lineage. Further morphological classification was not attempted The cells in Panel F have morphological characteristics of basophilic erythroblasts or earlier stages of erythroid cells (red dots in Figure 4).

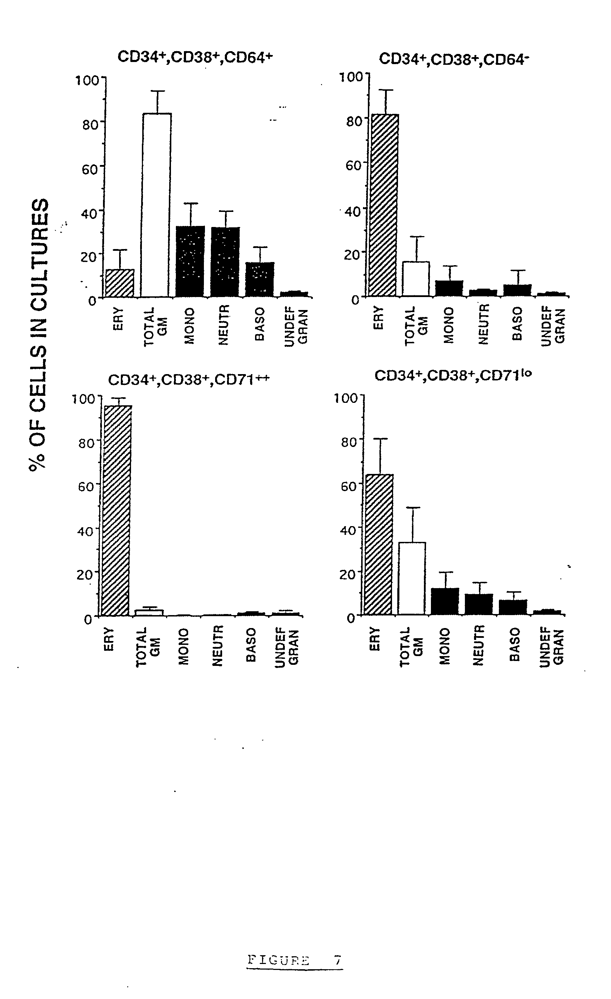

Figure 7 presents the frequency of erythroid and various granulo-monocytic cells in the progeny of sorted subsets of CD34+CD38+ cells after 6-8 days in liquid culture. Cells from the cultures were stained and analyzed as described in the legend to Figure 4. The dashed and solid black bars show the mean relative frequency of each of the populations described in Figure 4. Ery: erythroid cells, mono: monocytes/macrophages, neutr: neutrophilic granulocytes and precursors, baso: basophilic granulocytes, undef gran: undefined granulocytic cells. The open bar shows the mean percentage of granulo-monocytic cells (total GM) corresponding to the sum of the black bars. Error bars show standard deviations of six experiments, except for data for the CD33+ population, which represent three experiments. The frequency of cells that were not classified in any of these populations was always lower than 5%.

### Detailed Description of Invention

The instant invention is that CD64 is an early and specific marker of granulo-monocytic lineage commitment of hematopoietic progenitor cells. CD64 is undetectable on non-committed progenitor cells (CD34++, CD38-1lo, HLA-DR+) and is expressed on a subset of lineage-committed progenitors (CD34+) with higher orthogonal light scatter than the remaining CD34+ cells. CD64 is apparently expressed only on bone marrow and blood cells with light scatter properties and phenotypes typical for granulo-monocytic cells, and it is postulated that CD64 expression does not occur on cells outside the granulo-monocytic lineage. By way of comparison, the immunophenotypic markers, CD 13 and CD33 are not myeloid specific, as both were detected on non-committed progenitors. CD15 showed lower expression on a smaller subset of the CD34+ cells than CD64, indicating its comparative lack of utility as an early lineage marker. The specificity of CD64 for granulo-monocytic progenitors is confirmed by demonstrating high purity of granulomonocytic cells in the progeny of cultured CD34+,CD64+ cells, which cultures include that basophils as well as monocytes and granulocytes were present in the cultures from CD34+, CD64+. This further suggests that the antigen is expressed on most types of granulomonocytic progenitors.

Colony assays showing that CFU-GEMM were found only among progeny from CD64- HPC suggest that cells expressing CD64 are no longer multipotential. While a given number of CD64- cells give rise to more granulo-monocytic progeny than do the same number of CD64+ cells; this same number of CD64-cells also gave rise to a large number of erythroid cells. Thus, it is postulated that CD64+ granulo-monocytic progenitor cells have lost some degree of proliferative potential compared to their CD64-counterparts. Still, it is believed that CD64 is expressed at an early stage in granulo-monocytic differentiation, as the antigen seemed to appear before CD45 RA.

Lineage markers are frequently used to deplete bone marrow of mature cells during stem cell purification. The results showing staining of mAbs to CD13 and CD33 on non-committed HPC may therefore have important practical implications, as these markers are often included in mixtures of antibodies used for negative selection of primitive progenitor cells. It, therefore, seems likely that antibodies against CD33 may have similar effects. The negative staining of non-committed HPC with six different CD64 mAbs suggests that they may be useful in negative selection of stem cells.

Surface molecules with lineage-restricted expression on CD34+ cells may be involved in the control of early hematopoiesis. It is therefore interesting that CD64 is a receptor with a well-defined ligand and signal capacity. On monocytes and activated granulocytes CD64 functions as a high affinity IgG Fc receptor (FcgR), mediating phagocytosis and antibody dependent cellular cytotoxicity.

In summary, this invention presents CD64 as a granulo-monocytic lineage marker for CD34+ cells. CD64 antibodies therefore represent useful tools for identification and purification of progenitor subsets.

### Examples

The following examples present certain preferred embodiments of the instant invention, but are not intended to be illustrative of all embodiments.

### Antibodies:

All antibodies used in these examples are available from commercial sources. The antibodies were conjugated to fluorescein isothiocyanate (FITC), phycoerythrin (RPE), peridinin chlorophyll protein (PerCP), allophycocyanin (APC) or biotin via conjugation methods known in the art.

### Cell Staining Procedures:

For indirect immunofluorescence staining, nucleated bone marrow and blood cells were incubated with an unlabeled mAb, washed once in PBS-FCS and stained with fluorochrome-conjugated secondary antibodies. Before additional labeling with fluorochrome-conjugated mAbs the cells were washed once and incubated for 20 min. with 30% mouse serum. In some experiments biotin-conjugated mAbs were used, and labeling was followed by a final incubation step with fluorochrome-conjugated streptavidin. When only directly fluorochrome-conjugated mAbs were used (direct staining), they were all added simultaneously. All antibody-stainings were performed on ice, and each incubation step lasted 25 minutes. Immunostained cells were either kept on ice and analyzed immediately by flow cytometry or fixed in 0.5% paraformaldehyde and analyzed within 48 hours.

### Flow cytometric analysis:

Stained cells were analyzed using an experimental high-sensitivity flow cytometer, equipped with three lasers; a 488nM Argon Ion laser (15mW, for excitation of PerCP and FITC), a 633nm Helium-Neon laser (10mW, for excitation of APC or Cy5) and a 532nm YAG laser (20mW, for excitation of PE). For each sample 10,000 - 30,000 events were acquired. Forward light scatter, orthogonal light scatter and four fluorescence signals were determined for each cell.

### Cell sorting and culture:

Immunostained cells were sorted using a FACSVantage (Becton Dickinson) flow cytometer equipped with an Automated Cell Deposition Unit. FITC and PE fluorescence was excited by a 488nm Argon Ion laser and APC or Cy5 fluorescence by 90mW Spectrum laser tuned at 647nm. To eliminate doublets and cell aggregates, a pulse processor module was used on the forward light scatter parameter. In addition, sorting gates were set to exclude cells with large orthogonal and forward light scatter. The gates defining cells staining positively with CD64 and CD71 were set to include less than 2% of the cells stained with isotype control antibodies. The cells positive for CD71 were sorted as a bright (++) and a dim (+) population. The border between these two populations, were set based on visualization of cell clusters on dual parameter dot plots. All gates defining negatively staining populations were set back-to-back with the gates for the corresponding positive subset.

For liquid cultures, 2000-7000 cells were sorted per well in 24-well plates for culture. The wells contained myeloid long-term culture medium supplemented with 12.5% horse serum, 12.5% fetal bovine serum, 10⁻⁴ M 2-mercaptoethanol, 2 mM L-glutamine, 0.2 mM 1-inositol and 20 µM folic acid. The medium was supplemented with growth factors at final concentrations of 10 ng/ml recombinant human (rh) GM-CSF, 10 ng/ml rhIL-3, 500 U/ml rhIL-6, 10ng/ml rhlGF-1, 2.5 ng/ml rhbFGF, 40 ng/ml rhSCF.

For colony-assays, 10 or 100 cells were sorted per well in 96-well plates; 40 or 80 wells per sorted population. Wells contained 8 parts ISCOVE's methylcellulose ready-mix without growth factors, supplemented with 2-mercaptoethanol, FCS and BSA, diluted with 3 parts growth factors as recommended by the manufacturer. Final concentrations of growth factors were identical to those described above After sorting, plates were centrifuged for 7 minutes at 1000 rpm. All cultures were incubated in 5% CO₂ in air at 37°C.

After 6-8 days of culture, cells in liquid medium were detached from the wells by vigorous pipetting following 15 minutes incubation at 20°C with PBS-FCS containing 2 mM EDTA. A sample was taken out and mixed with a pellet consisting of 30,000 freezedried fluorescent latex beads (research reagent, BDIS) for cell counting by flow cytometry. After two washing steps the remaining cells were stained with antibodies and analyzed by flow cytometry as described above. To allow for discrimination and exclusion of dead cells, samples were incubated with propidium iodide at a final concentration of 1µg/ml.

In two experiments, subsets of immunostained, cultured cells were sorted and cytospin preparations were made. Cell morphology was examined after staining with Wright-Giemsa.

Plates with cells cultured in semisolid medium were scored at day 14 of culture to determine the differential content of myeloid (CFU-GM), erythroid (BFU-E and CFU-E) and multilineage (CFU-GEMM) colonies derived from each sorted fraction.

### Example 1

### Identification of potential myeloid-specific lineage markers within the CD34+ population.

Fluorochrome-conjugated antibodies used to detect progenitor subsets included CD34 PerCP, CD38 APC and HLA-DR FITC. Staining of the cells with potential lineage markers was detected using antibodies and goat-anti mouse IgG or goat anti-mouse IgM PE. Potential lineage-specific markers were identified as mAbs with undetectable staining of the non-committed progenitors and restricted staining among the lineage-committed progenitors. Within each lineage, the marker being expressed on the highest fraction of lineage committed cells was regarded as the earliest lineage-specific marker. Examples are shown in Figure 1 showing staining of CD34+ cells only.

Two antigens frequently referred to as markers of myeloid commitment, CD13 and CD33, were homogeneously expressed on the non-committed HPC in both fetal (Figure 1, panels D and E) and adult bone marrow. These results were reproduced both with directly conjugated mAbs against each molecule, as well as with two additional indirectly labeled CD33-mAbs with different epitope-specificity. The staining of CD13 and CD33 was bright enough to be detected on a standard FACSVantage configuration. The data in Figure 1, panels D and E therefore suggest that these two antigens are down regulated during lymphoid commitment rather than being de novo expressed on myeloid differentiated cells. On the average 31±14% of the CD34+, CD38+ FBM cells stained positively with CD33.

Two of the other markers tested fulfilled the criteria of potential lineage-specificity. The myeloid marker CD15 was not detected on non-committed HPC (black dots) and was positive on a subset of the CD34+,CD38+ cells (grey dots, Figure 1, panel A). A similar staining pattern was observed for mAbs to CD64 / FcgRI, Figure 1, panel B. In all experiments CD64 was expressed on at least twice as many of the CD34+,CD38+ cells as CD15 (average 23±11% versus 11±6%, n=4). The CD64 mAbs also stained more brightly than the CD15 mAbs (Figure 1), resulting in better separation between positive and negative cells. CD64+ cells had higher orthogonal light scatter than the majority of the CD64- cells, suggesting that the antibody stains predominantly myeloid progenitors (Figure 1, panel C). Similar results were obtained using six different antibodies to CD64, and no significant difference was seen when one antibody was used in indirect immunofluorescence or as PE- or APC-conjugates (not shown). The staining of CD34+,CD38+ cells with CD64 mAbs was sufficiently bright to be detected with standard FACSVantage configuration.

Most of the other mAbs investigated were excluded as lineage-specific markers because they stained non-committed HPC as a homogeneous positive population. These included CD11a/CD18 (LFA-1),CD29 (β1), CD31, CD43, CD44, CD45, CD48, CD49 (a-4), CD49e (a-5), CD5O (ICAM-3), CD52, CD53, CD54 (ICAM-1), CD58, CD59, CD63, TAPA-1 (CD81), CD99 (E2) and CD105 (endoglin) (not shown). Antibodies to CD62L (L-selectin), CD15s (sLex) and CD32/FcyRll stained both non-committed and committed progenitors heterogeneously (not shown). A few antibodies, including CD11b, CD11c, and CDw17, stained subsets of the CD34+ cells too variable and weak to be classified as clearly positive or negative (not shown). Antibodies that stained all HPC close to isotype control levels included CD14 (Figure 1, panel F), CD16, CD66b CD62E (E-selectin), CD62P (P-selectin) and VCAM-I (CD106).

Although there were some differences between adult and fetal bone marrow cells in staining intensities with some antibodies, the general patterns were the same.

The immunophenotyping results indicated that CD64 was the earliest and most specific myeloid lineage marker for CD34+ cells in fetal and adult bone marrow.

### Example 2

### Phenotypic characterization of CD64+ hematopoietic progenitor cells.

The distribution of CD64 on subsets of CD34+ HPC was investigated by costaining with CD45RA and CD71. The latter two antigens are absent or dimly expressed on non-committed CD34+ HPC. Erythroid cells are CD34+,CD45RAlo,CD71++, whereas granulo-monocytic cells are CD34+CD45RA+,CD71lo (Figure 2, panel A). In Figure 2 the CD34+,CD64+ cells are shown as black dots. The majority of these cells were CD45RA+,CD71 lo, suggesting that they may belong to the granulomonocytic lineage (n=3). The remaining CD34+,CD64+ cells (21.7±1.6% n=3) were CD45RAlo,CD71lo (Figure 2, panel C). This population constituted 41.5±14.3% (n=3) of the CD34+,CD45RAlo,CD71lo subset, which also contains the non-committed HPC (Figure 2, panel A). These results suggest that CD64 is expressed at an earlier stage than CD45RA. Figure 2 also shows that a large fraction of the CD34+CD45RA+,CD71lo cells (grey dots, Figure 2, panel A) were negative for CD64 (Figure 2, panel C). These cells most likely represent B cell progenitors, as more than 90% of the CD34+, CD45RA+CD64-cells stained positively with CD19 (n=3, not shown). As shown in Figure 3, CD64 and CD19 were mutually exclusive markers for CD34+ as well as CD34- bone marrow cells. The results from the immunophenotyping therefore suggest that CD64 has unique properties as a granulomonocytic lineage marker for CD34+ cells.

### Example 3

### Expression of CD64 on differentiating myeloid cells.

The expression of CD64 during the later stages of granulocyte and monocyte maturation was investigated in co-staining experiments with mAbs to CD14 and CD11b, which are unregulated during myeloid differentiation. Figure 4 shows the correlated staining of CD11b, CD 14 and CD64 on the total population of nucleated bone marrow cells, and the maturation pathways of granulocytes and monocytes are indicated with arrows. CD14+ monocytic cells are depicted black and showed high levels of CD64 throughout their maturation The majority of the remaining CD64+ cells represent granulocytic cells (grey dots). In this population, there was a decrease in the CD64 staining on CD11b cells, suggesting a down regulation of the antigen during terminal granulocyte differentiation. The CD64 staining remained, however, above isotype control levels on the most mature granulocytic FBM cells, as shown in Figure 3, panel A. Furthermore, staining of peripheral blood leukocytes with six different CD64 mAbs was negative on lymphoid cells, bright on monocytes and weakly positive on granulocytes. The mean fluorescence intensities of the different CD64 mAbs was on average 141.4-fold higher than isotype controls for monocytes (range 62.6-220.7) and 7.1 - fold higher for granulocytes (range 2.9 - 13.3). Expression of CD64 on unactivated mature granulocytes could be detected due to the high sensitivity instrument, yielding six times the sensitivity obtained with standard FACScan configuration, as described in materials and methods. The results from the immunophenotyping therefore indicate that CD64 is an early and specific marker of granulo-monocytic commitment that is expressed throughout the maturation of these cells. The marker was furthermore restricted to granulo-monocytic cells as later stages of erythroid cells (CD34-,CD71++) were negative for CD64.

### Example 4

### Lineage assessment of the progeny of cultured CD34+.CD38+ HPC by colony forming assays.

Fractions of CD34+, CD38+ fetal bone marrow cells labeled with mAbs to CD64 were sorted and cultured in semisolid medium. Ten or hundred cells were sorted into each well of 96-well plates containing medium with methylcellulose supplemented with SCF, Epo, GM-CSF, IL-3, IL-6, b-FGF and IGF, as described in materials and methods. Differential content of lineage-committed progenitors in each sorted fraction was determined by scoring the progeny for colony formation at day 14 of culture. In four experiments the CD34+,CD38+,CD64+ cells gave rise to 98±2% CFU-GM, 2±2% CFU-E / BFU-E and 0% CFU-GEMM. The CD34+, CD38+, CD64- cells gave rise to 28±12% CFU-GM 63±15% CFU-E / BFU-E and 9±4% CFU-GEMM (n=4).

### Example 5

### Differentiation of CD34+,CD38+ subsets in liquid culture assessed by flow cytometry.

Fetal bone marrow cells were stained, sorted and cultured as described for the assessment of cell expansion. After cell counting, the remaining cells were stained with CD14, CD15, CD50 and CD71 and analyzed by flow cytometry. This combination of antibodies was found to be useful to discriminate between granulocytic, monocytic, basophilic as well as erythroid cells in the cultures.

A typical example of the flow cytometric analysis of the progeny of CD34+, CD38+, CD64+ cells after 6 days of culture is illustrated in Figures 5, panels A and B. Based on the staining patterns, five predominant populations were defined and assigned different colors (Figure 5). In two experiments each of the five cell populations from cultured CD34+, CD38+, CD64+ cells was sorted and examined for cell morphology Examples are shown in Figure 6. The CD14-,CD15++,CD5O+,CD71^{dim} cells are depicted blue in Figure 5. They showed morphological characteristics of myeloblasts (10%), promyelocytes (40%) and later stages of neutrophilic granulocytes (50%), Figure 6, panel A. The CD14-CD15+,CD50+,CD71^{dim} subset is colored yellow in the figure and consisted almost entirely of cells with morphological appearance of basophilic granulocytes (97%), Figure 6, panel B. The CD14+,CD50+,CD71^{-->+} cells are depicted green in Figure 5. These were sorted as two populations, one CD14+,CD15++ and one CD14++,CD15+ population. The CD14⁺,CD15⁺⁺ population consisted predominantly of cells with characteristics of small monocytes/macrophages with fine granules in the cytoplasm (Figure 6, panel C). In contrast, the CD14⁺⁺,CD15⁺ population contained mainly large macrophage-like cells with an abundance of cytoplasmic vacuoles (Figure 6, panel D). This suggests that the CD14⁺,CD15⁺⁺ population represents an earlier stage of monocyte maturation as compared with the CD14⁺,CD15⁺⁺ population. The small population of CD14⁻,CD15⁻,CD50⁺,CD71⁻ cells is colored light blue and consisted of cells with an irregularly shaped nucleus. Due to the granular cytoplasm and high levels of CD50, they were classified as belonging to the granulocytic lineage, Figure 6, panel E. The CD14⁻,CD15^{lo},CD5O⁻,C071⁺⁺ subset is depicted red in Figure 5, and consisted almost entirely of erythroid cells (97%). The majority appeared to be basophilic erythroblasts or earlier stages, Figure 6, panel F.

Examples of the same flow cytometric analysis is shown for progeny of CD34+, CD38+, CD64- cells and CD34+, CD38+, CD71++ cells in Figure 5, panels C-F. The plots in Figure 5, panels A-F illustrate that progeny from different fractions of CD34+,CD38+ cells that were sorted and cultured. could be classified in the same five categories described above. The frequency of each population varied, depending on the progenitor subset sorted. The relative frequencies of the 5 populations was therefore determined after culture of all the sorted fractions of CD34+, CD38+ cells, including CD64+, CD64-, CD71++ and CD71+, as illustrated in Figure 7. Cultured CD34+,CD38+,CD64+ cells gave rise to the highest percentage of granulo-monocytic cells, in total 83.2±10.4%. The several hundred-fold larger expansion seen for erythrocytic compared to granulo-monocytic cells after the culture period indicates that the frequency of granulo-monocytic progenitors in the sorted CD34+,CD38+,CD64+ population was substantially higher than that of granulo-monocytic cells at day 6-8. In contrast, progeny from the CD34+,CD38+,CD64- cells contained 81.4±11% erythroid cells. The highest frequency of erythroid cells, 95.4±3.4%, was obtained from cultured CD34+,CD38+,CD71++ cells. As described above the CD34+,CD38+ CD33-cells did not expand in culture. The sorted CD34+,CD38+,CD33+ were thus representative for all myeloid and erythroid cells. The progeny of these cells contained 76.9±5.1% erythroid and 19.1±2.0% granulo-monocytic cells.

Unsorted nucleated FBM cells were stained and analyzed identically to the cultured cells, as shown in Figure 4, panels G and H. The lymphoid cells in the unsorted bone marrow were colored grey and identified as CD14-,CD5O+,CD15-, CD71- The frequency of erythroid and granulo-monocytic cells within the myeloid population was 29,5±7,8% and 70,5±7,8%, respectively (n=3). The cells from the cultures identified as basophils (yellow) and unclassified granulocytic cells (light blue) were, however, were not detected as unique populations among freshly isolated bone marrow cells.

The data from the functional analysis of CD38+,CD34+ subsets further support that CD64 is specific for several types of granulomonocytic progenitors within the CD34+ population.

It is apparent that many modifications and variations of this invention as herein set forth may be without departing from the spirit and scope hereof. The specific embodiments described are given by way of example only and the invention is limited only by the terms of the appended claims.

## Claims

1. A method of distinguishing granulomonocytic progenitor cells in a population containing CD34⁺ cells, comprising:
contacting said cells with antibodies specific for CD34 and CD64; and then flow cytometrically detecting cells that are CD34⁺CD64⁺.

2. A method of isolating granulomonocytic progenitor cells from a population containing CD34⁺ cells, comprising:
contacting said cells with antibodies specific for CD34 and CD64; and then flow cytometrically isolating cells that are CD34⁺CD64⁺.

3. The method of claim 1 or 2, wherein at least one of said antibodies is detectably and discriminably labelled by direct conjugation to a fluorochrome.

4. The method of any one of claims 1 - 3, wherein at least one of said antibodies is indirectly detected using a fluorochrome-conjugated secondary antibody.

5. The method of claim 1 or 2, wherein at least one of said antibodies is conjugated to biotin, and said biotin moiety is detected using fluorochrome-conjugated streptavidin.

6. The method of any one of claims 3-5 wherein said fluorochrome is selected from the group consisting of fluorescein isothiocyanate, phycoerythrin, peridinin chlorophyll protein, and allophycocyanin.

7. The method of claim 1 or 2 wherein said population of cells is selected from the group consisting of adult bone marrow, fetal bone marrow, fetal liver, cord blood, and peripheral blood.

## Patentansprüche

1. Verfahren zur Unterscheidung granulomonozytärer Progenitorzellen in einer CD34⁺-Zellen enthaltenden Population, umfassend:
Kontaktieren genannter Zellen mit für CD34 und CD64 spezifischen Antikörpern; und dann flowzytometrischer Nachweis von Zellen, die CD34⁺CD64⁺ darstellen.

2. Verfahren zur Isolation granulomonozytärer Progenitorzellen aus einer CD34⁺-Zellen enthaltenden Population, umfassend:
Kontaktieren genannter Zellen mit für CD34 und CD64 spezifischen Antikörpern; und dann flowzytometrische Isolation der Zellen, die CD34⁺CD64⁺ darstellen.

3. Verfahren nach Anspruch 1 oder 2, worin mindestens einer von genannten Antikörpern durch direkte Konjugation an ein Fluorochrom nachweisbar und diskriminierbar markiert ist.

4. Verfahren nach einem der vorangehenden Ansprüche 1 - 3, worin mindestens einer von genannten Antikörpern unter Verwendung eines Fluorochrom-konjugierten sekundären Antikörpers indirekt nachgewiesen wird.

5. Verfahren nach Anspruch 1 oder 2, worin mindestens einer von genannten Antikörpern an Biotin konjugiert ist und genannter Biotinteil unter Verwendung von Fluorochrom-konjugiertem Streptavidin nachgewiesen wird.

6. Verfahren nach einem der Ansprüche 3 - 5, worin genanntes Fluorochrom aus der Gruppe ausgewählt ist, die aus Fluoresceinisothiocyanat, Phycoerythrin, Peridininchlorophyllprotein und Allophycocyanin besteht.

7. Verfahren nach Anspruch 1 oder 2, worin genannte Zellpopulation aus der Gruppe ausgewählt ist, die aus reifem Knochenmark, fetalem Knochenmark, fetaler Leber, Nabelschnurblut und peripherem Blut besteht.

## Revendications

1. Une méthode permettant de distinguer les cellules progénitrices granulo-monocytaires dans une population qui contient des cellules CD34⁺, comprenant:
la mise en contact desdites cellules avec des anticorps dirigés spécifiquement contre les cellules CD34 et CD64; puis
la détection, par cytométrie en flux, des cellules qui sont CD34⁺CD64⁺.

2. Une méthode permettant d'isoler les cellules progénitrices granulo-monocytaires dans une population qui contient des cellules CD34⁺, comprenant:
la mise en contact desdites cellules avec des anticorps dirigés spécifiquement contre les cellules CD34 et CD64; puis
la détection, par cytométrie en flux, des cellules qui sont CD34⁺CD64⁺.

3. La méthode selon la Revendication 1 ou 2, dans laquelle un au moins desdits anticorps est marqué de façon détectable et différentiable par conjugaison directe avec un fluorochrome.

4. La méthode selon l'une quelconque des Revendications 1 à 3, dans laquelle un au moins desdits anticorps est détecté indirectement en utilisant un anticorps secondaire conjugué à un fluorochrome.

5. La méthode selon la Revendication 1 ou 2, dans laquelle un au moins desdits anticorps est conjugué à la biotine, ledit conjugué biotinylé étant détecté en utilisant une streptavidine conjuguée à un fluorochrome.

6. La méthode selon l'une quelconque des Revendications 3 à 5, dans laquelle ledit fluorochrome est sélectionné dans le groupe consistant en l'isothiocyanate de fluorescéine, une phycoérythrine, la protéine liant la péridinine-chlorophylle et l'allophycocyanine.

7. La méthode selon la Revendication 1 ou 2, dans laquelle ladite population de cellules est sélectionnée parmi un groupe consistant en la moelle osseuse adulte, la moelle osseuse foetale, le foie foetal, le sang du cordon ombilical et le sang périphérique.
